# EUROPEAN PATENT APPLICATION

(11) **EP 4 382 102 A1**
(43) Date of publication of application: **12.06.2024**
(21) Application number: 22212301.0
(22) Date of filing: 08.12.2022
(51) Int. Cl.: A61K 31/352, A61K 36/185, A61P 17/02

(54) **COMPOSITIONS FOR USE IN POST-OPERATIVE PAIN AND INFLAMMATION TREATMENT**

(71) Applicant: UAB Fitodenta, Kaunas (LT)
(72) Inventor: iurkus, Juozas, Vilnius (LT); Puisys, Algirdas, Vilnius (LT); Mileris, Edvinas, Vilnius (LT)
(74) Representative: Petniunaite, Jurga

(57) **Abstract**

Compositions for use in treatment of post peri-orthodontal, post oral cavity surgery condition including pain and inflammation related swelling is taught. The compositions are plant-based per oral spray and an oleo gel topical composition. The usage of both components provides reduce pain and inflammation related swelling for all patient groups without emergence of bacterial infection.

## Description

### FIELD OF THE INVENTION

The invention relates to plant-based compositions for use in methods of topical and oral treatment for post-operative and acute pain of the mouth.

### BACKGROUND OF THE INVENTION

All surgical interventions create an acute wound which results in trauma causing acute inflammation conditions which usually is accompanied with swelling of soft tissue in the area of surgical procedure and intensive pain. Mentioned clinical conditions are treated using oral and or topical formulations of non-steroid ant -inflammatory drugs (NSAID) or strong pain reduction features possessing substances based on opioid related active pharmaceutical ingredient (API). In addition, during the healing course patients are exposed to the risk of bacterial infection related complications which are usually controlled by various courses of antibiotics.

Utilization of mentioned therapies for treatment of post-surgical procedures elucidates a certain risk factors which might be related to a health/life threating complications: (i) Use of NSAIDS - heart, kidney failure, internal bleeding; Use of opioids - drug addictions, overdose, (iii) antibiotics -proliferation of pathogen resistance against antibiotics. Thus, an alternative option for treatment of post-surgical inflammation conditions are required.

Oral surgical wounds heal in a very similar way, soft tissue healing is somewhat conditioned by that of the underlying bone tissue. First intention bone healing occurs in correctly repositioned and perfectly stabilized fractures, while secondary intention healing occurs when a bone defect has to be spontaneously filled, as in extraction sockets or in other post-surgical residual bone cavities or gaps. A particular form of bone healing is that which occurs at the peri-implant surface level and can be defined as early or late stage healing. Early stage healing is that of a foreign body response and is influenced by implant stability and implant surface morphology and material. Both contact and distance osteogenesis allow to fill the gap between the host bone and the implant surface in this stage, resulting in immature woven bone. Late stage bone healing involves a remodelling process of both the host and immature bone which leads to the formation of mature lamellar bone which continues throughout life since it is significantly influenced by mechanical forms of stress.

Soft tissue painless swelling usually occurs at the surgical site from the second day. This swelling is therefore directly proportional to the extension and duration of the procedure and it tends to progressively and spontaneously decrease from the 3rd or 4th day on. Slight swelling and redness are common at the insertion point of sutures, usually more pronounced in the vestibular mucosa than in the ridge area and sometimes associated with a small area of ulceration. Swelling is somewhat unusual at distant locations such as the ipsilateral cheek and sub-mandibular region, due to edema and inflammation diffusion from the surgical site caused by surgical trauma. Rarely, sub-mandibular lymph nodes may swell and be painful on palpation. A slight rise in temperature may also occur for a few hours after surgery due to transient bacteremia. Light bleeding is common for the first few hours after surgery but it may occur after hours or continue for 2-3 days due to suture loosening, flap instability or dehiscence. Ecchymoses and hematomas may occur after 2-4 days and are caused by blood escape from the surgical site into the sub-mucosal and, rarely, into the sub-cutaneous soft tissues.

C. sativa produces many pharmacologically active secondary metabolites including cannabinoids, terpenes and flavonoids that share anti-inflammatory, antioxidant antimicrobial, analgesic, anti-cancer, anxiolytic properties. In medical systems, C. sativa, was utilized for toothache management, though it is also likely that the plant may have also been used in the treatment and prevention of dental caries and reduction in gum inflammation. There is currently a wide range of cannabinoid-based oral products currently on the market, and studies being conducted on cannabinoid-based pharmaceuticals in general, are increasingly positive and evidence-based. These products include cannabidiol (CBD) capsules, CBD pills, CBD Hemp Oil Tinctures, CBD Oil, CBD-infused toothpastes, CBD Oral Sprays, CBD-infused mouthwashes, CBD chewing gum and even CBD-infused dental fillings.

The aforementioned products are primarily used as analgesics to provide relief from tooth pain and gum soreness, and as anti-microbials and anti-septic agents to maintain oral hygiene, and anti-inflammatory agents to control inflammation of the gums. Due to antimicrobial resistance, it was then concluded that these cannabinoids may be a safer alternative to traditional synthetic oral hygiene products.

In one study by Vasudevan and Stahl ("Cannabinoids infused mouthwash products are as effective as chlorhexidine on inhibition of total culturable bacterial content in dental plaque samples". J. Cannabis Res. 2020, 2, 20), the efficacy of two mouthwash products, one containing a <1 % cannabidiol (CBD) per weight and another containing < 1% cannabigerol (CBG) per weight, respectively, were investigated against total-culturable bacteria from dental plaque samples. In comparison to chlorhexidine 0.2%, frequently used in traditional synthetic mouthwash, both cannabinoid-infused mouthwash products demonstrated very similar bactericidal efficacy.

By taking into account mentioned therapeutic features of C. Sativa cannabinoid-based pharmaceutical compositions there are several patents for the maintenance of general oral hygiene and for specific oral and dental diseases.

In U.S. Patent Publication 2020/0222361A1 (published 12 November 2020), cannabis-based composition comprising cannabis-extract, derivatives, and/or at least one synthetic cannabinoid intended for the treatment of dental pulp infection, pulp inflammation, dental (jaw) bone defects is taught.

In U.S. Patent No. US10172786B2 (first published 16 June 2016), cannabinoid-based oral care compositions (tooth paste, a tooth powder, or a mouthwash solution) for the treatment of oral infectious disease, including periimplantitis, periodontitis, oral mucositis, and dental pain are taught, wherein cannabinoid may be cannabidiol and/or cannabigerol.

In In South Korean Patent Publication KR20120133135A (published 10 December 2012), extract of C. sativa L. for use in toothpaste, oral cleanser, or oral spray for the treatment of dental caries is taught.

None of the above publications teach a compositions of cannabinoid-based oral spray and cannabinoid-based oleo gel for combined use in pain treatment of the mouth.

### SUMMARY OF THE INVENTION

The invention is a system for use in treatment of post-surgical pain and inflammation. The system is based on oral spray formulation and oleo gel topical composition. Both system components are formulated using combinations of the defined cannabis sativa substances-cannabinoids, terpenes and supplemented with defined herbal extracts. Defined cannabis compounds are combined with plant components to ensure pharmacological effects and formulation stability. The oral spray composition is used in perioral manner, daily up to 10 days in tandem with topical compositions which in all cases is applied only on the external facial area of surgical procedure. Topical composition is not applied directly on the surgical wound or in oral cavity and not applied on the closed surgical wounds.

The inventive system is aimed for therapeutic use for reduction of pain and inflammation related swelling of post peri-orthodontal, post oral cavity surgery condition. The system is comprised of defined per oral spray and topical composition, an oleo gel. Empirical clinical data showed that usage of both components provides therapeutic relief by reduction of pain and inflammation related swelling for all patient groups without emerging of bacterial infection. The best clinical results were observed only when both therapeutic components were used in tandem.

Clinical data showed that usage of herein described treatment system allowed to reduced usage of NSAIDS and antibiotic during the healing course.

### BRIEF DESCRIPTION OF THE DRAWINGS

FIG. 1 shows morphology of THP-1 cells.
FIG. 2 Levels of IL8 and IL6 secreted into THP-1 cell culture media and quantified by ELISA.
FIG. 3 shows pain level results from a first patient group study
FIG. 4 shows pain level results from a first patient group study.
FIG. 5 shows swelling results from a first patient group study
FIG. 6 shows swelling results from a first patient group study.

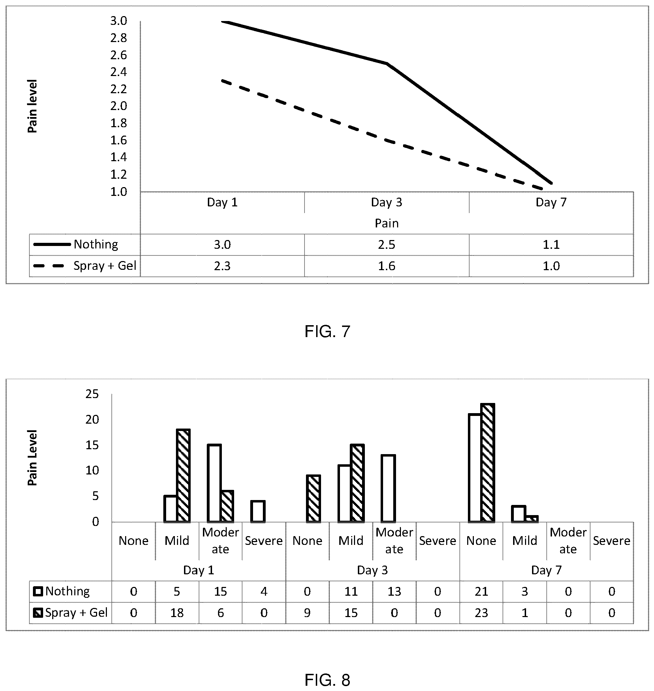

FIG. 7 shows pain level results from a second patient group study
FIG. 8 shows pain level results from a second patient group study.

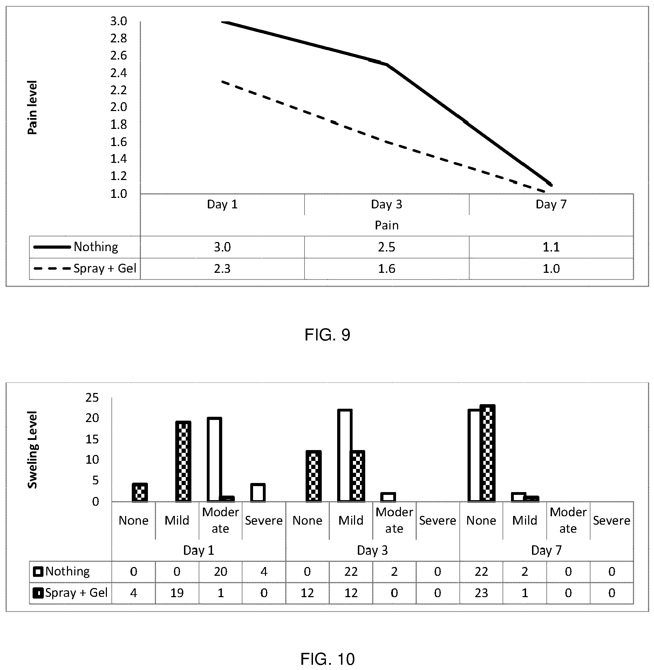

FIG. 9 shows swelling results from a second patient group study
FIG. 10 shows swelling results from a second patient group study.

### DETAILED DESCRIPTION OF THE INVENTION

In preferred embodiments, compositions of phyto-cannabinoids together with terpenes and additionally plant extracts are described.

In one embodiment, the composition comprises 1-10% cannabinoids from *C. Sativa,* less than 2 % mixture of terpenes, and 1-15% of plant extracts in a carrier excipient.

In another embodiment, the composition comprises 2-6% cannabinoids from *C. Sativa,* less than 1 % mixture of terpenes, and 2-10% of plant extracts in a carrier excipient.

In one embodiment, the composition comprises 2-6% crystallized cannabinoid isolates from *C. Sativa,* less than 1 % mixture of terpene isolates, and 2-10% of plant extracts in a carrier excipient.

In another embodiment, the composition comprises 2-6% cannabinoids *C*. *Sativa,* wherein cannabinoids are CBD and one or more cannabinoids are chosen from CBG, CBC, and CBN. The composition further comprises less than 1% mixture of terpenes, wherein the mixture of terpenes comprises one or more of beta-caryophyllene, myrcene, humulene, linalool, A-pinene, B-pinene, limonene-D, terpinolene, A-bisabolol, and others. The composition further comprising 2-10% plant extracts, wherein one or more plant extracts are chosen from calendula officinalis extract, syzygium aromaticum extract, mentha piperita extract, glycyrrhiza sp. extract, clove bud CO2 extract, rosemary antioxidant extract, peppermint leaf CO2 extract. In the example composition, the excpient is one or more of medium chail triglycerides (MCT) oil and fumed silica.

In one example, the composition is a liquid composition comprising 5-6% cannabinoids from *C. Sativa,* less than 1% mixture of terpenes, 5-10% plant extracts, and a carrier oil. The composition can be used as a liquid spray.

In another example, the composition is a liquid composition comprising 5-6% cannabinoids *C. Sativa,* wherein cannabinoids are CBD and one or more cannabinoids are chosen from CBD, CBG, CBC, and CBN, less than 1% mixture of terpenes, wherein the mixture of terpenes comprises one or more of beta-caryophyllene, myrcene, humulene, linalool, A-pinene, B-pinene, limonene-D, terpinolene, A-bisabolol, and others, 5-10% plant extracts, wherein one or more plant extracts are chosen from calendula officinalis extract, syzygium aromaticum extract, mentha piperita extract, glycyrrhiza sp. extract, clove bud extract, rosemary antioxidant extract, peppermint leaf extract, and MCT or cannabis sativa carrier oil.

An exemplary liquid composition is shown in Table 1.

| Table 1: Liquid composition 100 | |
|---|---|
| CBD | 5.1 % |
| CBG | 0.7% |
| Caryophyllene | 0.5% |
| Myrcene | 0.1 % |
| Humulene | 0.1 % |
| Calendula officinalis extract | 3% |
| Syzygium aromaticum extract | 3% |
| Mentha piperita extract | 0.5% |
| Glycyrrhiza sp.extract | 1% |
| MCT oil | 86% |

In another example, the composition is an oleo gel composition comprising 2-5% cannabinoids from *C. Sativa,* less than 1% mixture of terpenes, 2-10% plant extracts, fumed silica, and a carrier oil.

In yet another example, the composition is an oleo gel composition comprising 2-5% cannabinoids from *C. Sativa,* wherein cannabinoids are CBD and one or more cannabinoids are chosen from CBD, CBG, CBC, and CBN, less than 1% mixture of terpenes, wherein the mixture of terpenes comprises one or more of beta-caryophyllene, myrcene, humulene, linalool, A-pinene, B-pinene, limonene-D, terpinolene, A-bisabolol, and others, 2-10% plant extracts, wherein one or more plant extracts are chosen from calendula officinalis extract, syzygium aromaticum extract, mentha piperita extract, glycyrrhiza sp. extract, clove bud CO2 extract, rosemary antioxidant extract, peppermint leaf CO2 extract, fumed silica and MCT carrier oil.

An exemplary oleo gel composition is shown in Table 2.

| Table 2: Oleo gel composition **200** | |
|---|---|
| *Crystalized cannabinoid isolates:* | |
| Cannabidiol (CBD) | 2.00% |
| Cannabigerol (CBG) | 0.25% |
| Cannabichromene (CBC) | 0.25% |
| Cannabinol (CBN) | 0.0625% |
| | |

| *Terpene isolates:* | |
|---|---|
| Beta-Caryophyllene | 0.10% |
| Myrcene | 0.025% |
| Humulene | 0.025% |
| Linalool | 0.0125% |
| A-Pinene | 0.0125% |
| B-Pinene | 0.0125% |
| Limonene-D | 0.0125% |
| Terpinolene | 0.0125% |
| A-Bisabolol | 0.00625% |
| | |

| *Plant extracts:* | |
|---|---|
| Clove Bud CO2 Extract | 1.00% |
| Rosemary Antioxidant Extract | 1.00% |
| Peppermint Leaf CO2 Extract | 0.50% |
| | |

| *Excipients:* | |
|---|---|
| Medium Chain Triglycerides Oil | 84.719% |
| Fumed Silica | 10.00% |

The liquid composition is suitable for use in oral wound treatment by spraying into the mouth cavity around/on the affected area.

In one example, the liquid spray is suitable for administering by applying the liquid composition immediately after surgical procedure, within a10-60 minute time frame, of 3 to 4 sprays, each spray generating approximately 1 to 3 mL of liquid or generating less than 1 mL of liquid, into the oral cavity in the area of surgical wound. After application of the liquid spray composition, the mouth is not rinsed with any other liquid for at least 30 minutes. Spraying is repeated 3-8 times per 24 hours with the intervals of 6 to 8 hours. Alternatively, spraying is repeated 3-4 times per 24 hours with the intervals of 6 to 8 hours. Further alternatively, spraying is repeated 3-8 times per 24 hours with the intervals of 3 to 6 hours. The course should be continued 3 to 10 days, 3 to 7 days, or 3 to 5 days.

In another instance, the oleo gel composition is suitable for use in topical applications around the mouth.

In one example, the oleo gel is suitable for administering by applying to external tissue around the mouth or area of injury while not applying directly on to area of injury or open wounds.

In another example, gentle application of fine layer of an oleo gel composition, for example 0.5-2 g of oleo gel, in the facial area of surgical procedure is recommended. Oleo gel composition should be applied immediately, within a 10-60 minute time frame, after oral surgery. Application is repeated 3-8 times per 24 hours with the intervals of 6 to 8 hours. Alternatively, application is repeated 3-4 times per 24 hours with the intervals of 6 to 8 hours. Further alternatively, application is repeated 3-8 times per 24 hours with the intervals of 3 to 6 hours. The course should be continued 3 to 10 days, 3 to 7 days, or 3 to 5 days.

In yet another embodiment, the spray and gel are suitable for administering in tandem, the method comprising gentle application of fine layer of an oleo gel composition, for example 0.5-2 g of oleo gel, in the facial area of surgical procedure. Oleo gel composition should be applied immediately, within a 10-60 minute time frame, after oral surgery. Application is repeated 3-8 times per 24 hours with the intervals of 6 to 8 hours. Alternatively, application is repeated 3-4 times per 24 hours with the intervals of 6 to 8 hours. Further alternatively, application is repeated 3-8 times per 24 hours with the intervals of 3 to 6 hours. The course should be continued 3 to 10 days, 3 to 7 days, or 3 to 5 days. The method further comprising applying the liquid spray composition immediately after surgical procedure, within a 10-60 minute time frame, of 3 to 4 sprays, each spray generating 1 to 3 mL of liquid or generating less than 1 mL of liquid, into the oral cavity in the area of surgical wound. After application of the liquid spray composition, the mouth is not rinsed with any other liquid for at least 30 minutes. Spraying is repeated 3-8 times per 24 hours with the intervals of 6 to 8 hours. Alternatively, spraying is repeated 3-4 times per 24 hours with the intervals of 6 to 8 hours. Further alternatively, spraying is repeated 3-8 times per 24 hours with the intervals of 3 to 6 hours. The course should be continued 3 to 10 days, 3 to 7 days, or 3 to 5 days.

### EXAMPLES

**Example 1:** Evaluation performed of toxicity and efficacy and anti-inflammatory activity of liquid spray composition of Table 1, liquid composition **100,** for anti-inflammatory and pro-regenerative effects using models based on human monocytes (cell line THP-1).

### Material and methods.

Human monocytes (cell line THP-1) were cultured in RPMI medium with the addition of 10% fetal calf serum and antibiotics as described elsewhere. Cells were exposed to the liquid composition **100**, with carrier oil alone or dispersing agent ethanol (also added to the cultures). Effects were assessed after 24h.

### Material and Methods:

**Assays performed:** The liquid composition **100** was tested for its inflammation regulatory capacity. Cells were cultured conventionally or exposed to pro-inflammatory mediator interleukin-1beta (IL-1β (1ng/ml)) and then treated with the liquid composition **100.** Control cells were exposed to carrier oil or ethanol (used as a dispersing agent).

Two doses of the liquid composition **100** taken for experiments and was normalized by taking into account quantity of CBD within the liquid composition **100.** Accordingly, the cells were exposed with the appropriate amounts of liquid composition **100** to reach concentrations of CBD within culture media of 1 and 5 µg/ml, respectively.

### Results

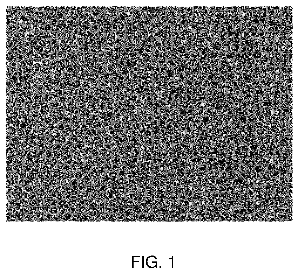

Cell viability under the influence of liquid composition **100**. Metabolic activity of THP-1 monocytes (**FIG. 1**) was assessed daily for a period of few days by means of PrestoBlue method as described in Aldo PB, et. al. ("Effect of culture conditions on the phenotype of THP-1 monocyte cell line". Am J Reprod. Immunol. 2013 Jul;70(1):80-6). For comparison a viability of untreated cells, cells exposed to carrier oil or ethanol was investigated as well.

PrestoBlue results demonstrate that there is no cytotoxic activity of any substances tested on the viability of THP-1 cells.

### Quantification of secreted interleukins' (IL6 and IL8) levels

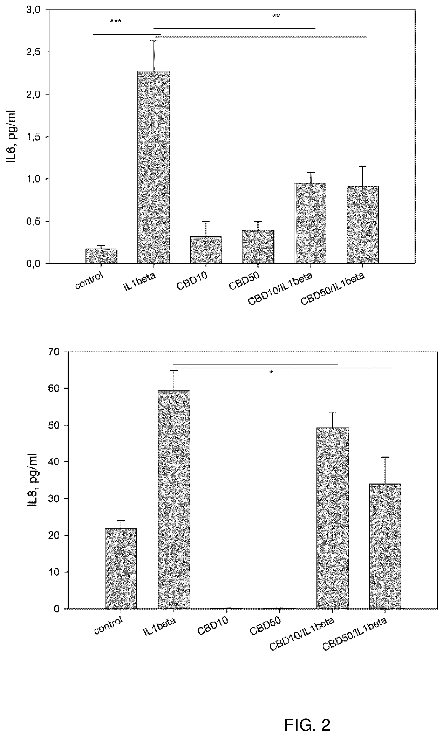

THP-1 were exposed to liquid composition **100** together with IL-1β (1ng/ml) or without it (**FIG 2**). Liquid composition **100** significantly reduced IL-6 and IL-8 production in IL-1β (1ng/ml)-exposed THP-1 cells.

The results show that liquid composition **100** is not cytotoxic towards human monocyte cell line THP-1. And further show that liquid composition **100** significantly reduces acute (24h) pro-inflammatory activation of monocytes, i.e. IL6 and IL8 production is significantly upregulated upon exposure to IL-1β, but liquid composition **100** ameliorates this activation back to the control level when added together with IL-1β.

### EXAMPLE 2

Clinical studies demonstrate the efficacy of usage of cannabinoid based oleo gel composition shown in Table 2, oleo gel composition **200**, and liquid composition **100** for reduction of inflammation and pain after minor (simple tooth extraction, implant placement) and major dental surgical procedures (bone regeneration, complicated third molar extraction)

### Materials and methods.

### Topical oleo gel usage:

External, gentle application of fine layer of the oleo gel composition **200** (require 0.5-2 g of oleo gel) in the facial area of surgical procedure. Oleo gel composition **200** was applied immediately (10-60 min time frame) after oral surgery. Application was repeated 3-4 times per 24 h with the intervals of 6 to 8 hours. The course was continued up to 5 days.

### Per oral usage of liquid spray composition:

Liquid composition **100** was applied immediately after surgical procedure (10-60 min time frame) (3-4 sprays, generates: < 1 mL of liquid) into the oral cavity in the area of surgical wound. After spraying with liquid composition **100**, the patients did not rinse mouth with any other liquid for at least 30 minutes. Spraying was repeated 3-4 times per 24 hours with intervals of 6 to 8 hours. The course was continued up to 5 days.

### Traditional treatment

Amoxicillin, at a dosage of 1000mg was administered twice per day, Ibuprofen, at a dosage of 400mg, or Nimesulidum, at a dosage of 100mg, was administered twice per day. A 0.12% Chlorhexidinum solution was used to rinse the mouth twice per day. The course was continued up to 5 days.

### Mixed treatment

Combinations of the above described Topical oleo gel usage, per oral usage of liquid spray composition, and traditional treatments were administer to patients in control studies.

In mixed traditional treatment and topical oleo gel, no modification to the usage were made.

In mixed traditional treatment and per oral usage of liquid spray composition, the 0.12% chlorhexidinum solution was not used.

In mixed traditional treatment with both topical oleo gel and per oral usage of liquid spray composition, the 0.12% chlorhexidinum solution was not used.

### Scope of Patient groups:

144 patients were involved in the first group and were divided in 4 subgroups:
1.1. Antibiotics only (36 patients) - referred as "Traditional Treatment"
1.2. Antibiotics + CBD oral spray only (36 patients);
1.3. Antibiotics + CBD oleo gel only (36 patients);
1.4. Antibiotics + CBD oleo gel and oral spray (36 patients).

Patients received big dental surgery procedures involving gingival flap elevations, flap releasing, bone regeneration, and complicated third molar extraction.

Antibiotics were prescribed to all groups and CBD products-oleo gel, oral spray or both-were additionally provided according to subgroups.

48 patients in the second group with following subgroups:
2.1. No "Traditional" treatment - no antibiotics, no NSAIDS (24 patients );
2.2. CBD gel and spray (24 patients ).

Patients received simple dental surgery-no flap releasing-and no additional medicine were prescribed for one group and oleo gel and liquid spray were administered to the other group.

Pain level, swelling, need for pain killers and primary/secondary healing was registered. Relative pain level of each patient was recorded after oral surgery and after receiving a prescribed treatment regimen. The pain was recorded on a scale of 1 to 4, 4 being the highest level of pain. Swelling was rated on a relative scale of 1 to 4, 4 being the most swelling.

### Results

### First group

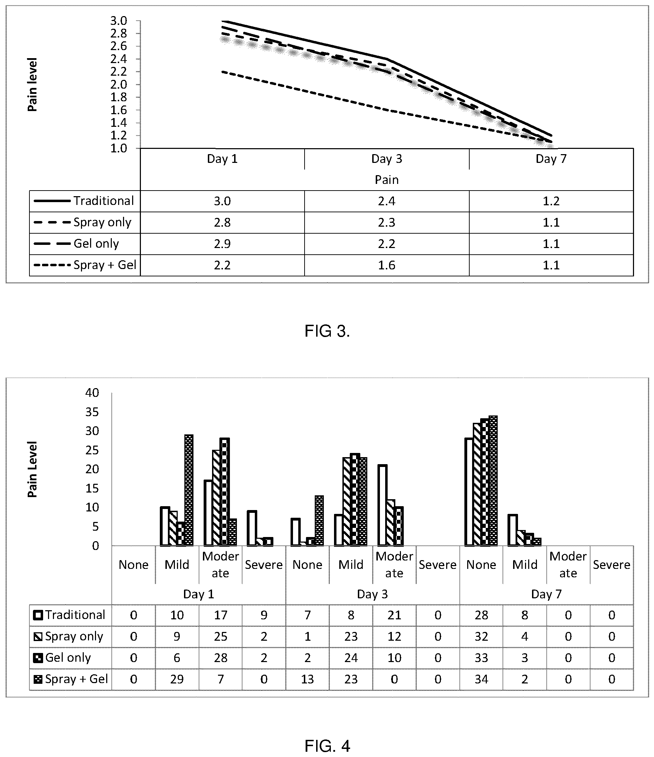

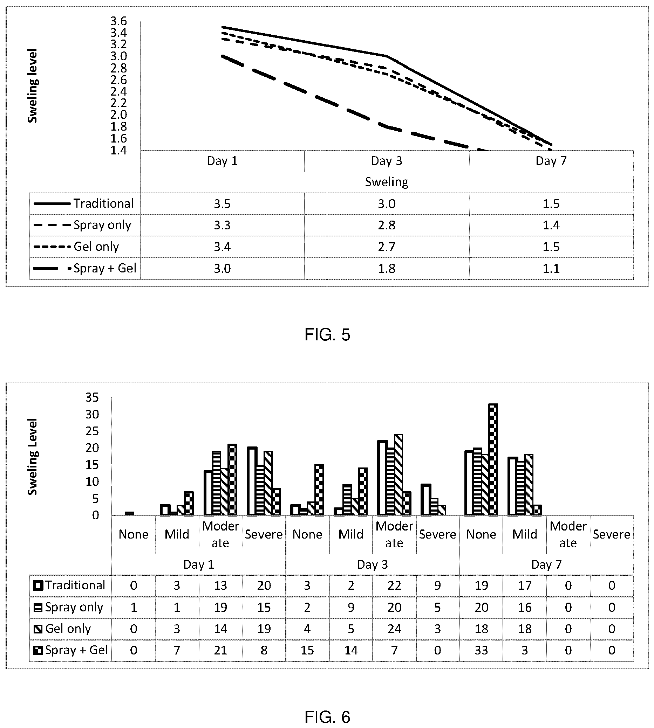

The results of the patient pain levels are shown in FIG. 3 and 4. The results of swelling are shown in FIG. 5 and 6. Combined usage of liquid composition **100** and oleo gel composition 200 showed the highest pain reduction score. The pain reduction is significant immediately after usage of the CBD-based therapeutic system.

Combination liquid composition **100** and oleo gel composition **200** treatment group showed statistically significant lowest level of swelling on day 1 as well as on day 3.

Indicated need for pain reducing drugs was lowest within the Combination liquid composition **100** and oleo gel composition **200** treatment group. Primary and secondary healing was not influenced and has no difference between groups.

### Second group

Nothing refers to "no Traditional treatment" with no antibiotics and no NSAIDS Combination liquid composition **100** and oleo gel composition **200** treatment group showed statistically significantly reduced swelling and pain during first week of healing.

The above examples show that the combination of cannabinoid based oleo gel and oral spray resulted in statistically significant reduction of inflammation caused pain and need for pain killers after minor and major dental surgery procedures. Usage of combination of cannabinoid based oleo gel is recommended after dental surgery procedures to reduce inflammation and increase healing capacity

## Claims

1. A composition comprising 1-10% cannabinoids from *C. Sativa,* less than 2 % mixture of terpenes, and 1-15% of plant extracts in a carrier excipient.

2. The composition of claim 1, wherein the cannabinoids are crystallized cannabinoid isolates from *C. Sativa,* and wherein the terpenes are terpene isolates.

3. The composition of claim 1, wherein cannabinoids are CBD and one or more cannabinoids are chosen from CBG, CBC, and CBN;
wherein the mixture of terpenes comprises one or more of beta-caryophyllene, myrcene, humulene, linalool, A-pinene, B-pinene, limonene-D, terpinolene, A-bisabolol, and others;
wherein one or more plant extracts are chosen from calendula officinalis extract, syzygium aromaticum extract, mentha piperita extract, *glycyrrhiza sp.* extract, clove bud CO2 extract, rosemary antioxidant extract, peppermint leaf CO2 extract; and
wherein the excipient is one or more of medium chain triglycerides (MCT) oil and fumed silica.

4. The composition of claim 1, wherein the composition is a liquid composition.

5. The composition of claim 4, comprising:
5-6% cannabinoids, wherein the cannabinoids are CBD and one or more cannabinoids are chosen from CBD, CBG, CBC, and CBN;
less than 1% mixture of terpenes, wherein the mixture of terpenes comprises one or more of beta-caryophyllene, myrcene, humulene, linalool, A-pinene, B-pinene, limonene-D, terpinolene, A-bisabolol, and others;
5-10% plant extracts, wherein one or more plant extracts are chosen from calendula officinalis extract, syzygium aromaticum extract, mentha piperita extract, glycyrrhiza sp. extract, clove bud extract, rosemary antioxidant extract, peppermint leaf extract; and
MCT or cannabis sativa carrier oil.

6. The composition of claim 1, wherein the composition is an oleo gel.

7. The composition of claim 6, comprising:
2-5% cannabinoids from C. Sativa, wheren cannabinoids are CBD and one or more cannabinoids are chosen from CBD, CBG, CBC, and CBN;
less than 1% mixture of terpenes, wherein the mixture of terpenes comprises one or more of beta-caryophyllene, myrcene, humulene, linalool, A-pinene, B-pinene, limonene-D, terpinolene, A-bisabolol, and others;
2-10% plant extracts, wherein one or more plant extracts are chosen from calendula officinalis extract, syzygium aromaticum extract, mentha piperita extract, glycyrrhiza sp. extract, clove bud CO2 extract, rosemary antioxidant extract, peppermint leaf CO2 extract; and
excipients fumed silica and MCT carrier oil.

8. The composition of claim 5 for use in method of treatment of post-operative oral wounds **characterized in that** the composition of claim 5 within 10-60 minutes after a surgical procedure by applying 3-4 sprays, each generating 1 to 3 mL of liquid into the oral cavity in the area of surgical wound, wherein after spraying with the composition, the patients does not rinse mouth with any other liquid for at least 30 minutes;
wherein treatment was repeated 3 to 8 times per 24 hours with intervals of 3 to 6 hours; and
wherein the course is administered for 3 to 10 days.

9. The composition of claim 7 for use in method of treatment of post-operative oral wounds **characterized in that** the composition of claim 7 is applied within 10-60 minutes after a surgical procedure by externally applying a fine layer of 0.5-2 g of the composition in the facial area of surgical procedure;
wherein treatment was repeated 3 to 8 times per 24 hours with intervals of 3 to 6 hours; and
wherein the course is administered for 3 to 10 days.

10. The composition of claim 5 and 7 for use in method of treatment of post-operative oral wounds **characterized in that** the composition of claim 5 is applied within 10-60 minutes after a surgical procedure by applying 3-4 sprays, each generating 1 to 3 mL of liquid into the oral cavity in the area of surgical wound, wherein after spraying with the composition, the patients does not rinse mouth with any other liquid for at least 30 minutes;
**characterized in that** the composition of claim 7 is applied within 10-60 minutes after a surgical procedure by externally applying a fine layer of 0.5-2 g of the composition in the facial area of surgical procedure;
wherein treatment was repeated 3 to 8 times per 24 hours with intervals of 3 to 6 hours; and
wherein the course is administered for 3 to 10 days.
